# EUROPEAN PATENT APPLICATION

(11) **EP 4 248 945 A1**
(43) Date of publication of application: **27.09.2023**
(21) Application number: 23162826.4
(22) Date of filing: 20.03.2023
(51) Int. Cl.: A61K 9/00, A61K 36/15, A61K 47/14, A61K 9/06, A61P 15/14, A61P 1/00

(54) **VETERINARY TOPICAL COMPOSITION FOR THE ANTIMICROBIC TREATMENT OF DAIRY ANIMAL UDDERS**

(30) Priority: 21.03.2022 IT 202200005462
(71) Applicant: Green Innovation GmbH, 6020 Innsbruck (AT)
(72) Inventor: Leonardi, Giuliano, ISOLA DOVARESE (CR) (IT)
(74) Representative: Villa, Livia

(57) **Abstract**

The present invention concerns a composition for antimicrobic treatment of udders of dairy animals. The composition is preferably to be administered during the dry off period, more preferably intramammary, for treating and/or preventing udder infections and mastitis.

## Description

### FIELD OF THE INVENTION

The present invention concerns a veterinary topical composition having antimicrobic efficacy, for treating udders of dairy animals. The composition is preferably administered during the dry off period, more preferably via intramammary route, for treating and/or preventing udder infections and mastitis.

### STATE OF THE ART

All dairy animals, in particular cows, require a period of time, prior to calving, where milk production is stopped, in order to prepare the udder for the next lactation and to allow the mammary tissues repair and regenerate. The process of stopping the milk production is known as "dry-of" or simply "dry" period. Typically, the duration of this period is between 40 and 70 days.

The dry period is a very important phase of a dairy animal's lactation cycle: any abnormalities, such as infections, during the dry period will have a negative effect on the animal's health and milk production after calving.

Where a teat is open during the dry period, bacteria can invade the udder where they can cause infection, provoking inflammation of udder (mastitis), affecting the next lactation period.

Approximately, 60% of all early lactation mastitis cases has an origin in the dry period. Also, a high volume of residual milk in the udder stimulates the white blood cells to concentrate on absorbing milk fat cellular debris so that white cells become less active in preventing bacteria from entering the udder.

When drying off an animal, the goal is then to suddenly end milk secretion and also to seal the teat canal as quickly as possible, to prevent bacteria from entering the teat canal during the dry period, thus preventing new infections from occurring prior to calving or immediately after calving, and to cure any existing infection.

Typically, a keratin plug is formed in the teat canal during the entire dry period as udder's natural defense mechanism. The plug then slowly disappears at the end of the dry off period in preparation of lactation. However, in some cases the plug doesn't form at all or forms weakly. For instance, cows with a high milk volume at drying off have a weaker keratin plug compared to other cows due to delayed keratin plug formation in the teat canal.

The use of internal teat sealants at drying off, to provide a sustained physical barrier in the teat cistern, thereby preventing the ingress of bacteria into the udder, is a well-established practice in dairy husbandry.

Typically, teat sealants are used in conjunction with an antibiotic intramammary infusion, which is administered immediately prior to the teat sealant.

An intramammary antibiotic treatment at drying off can prevent new infections during the first few weeks of the dry period and also eliminate existing infections. However, there are concerns about the use of an antibiotic, due to antimicrobial resistance. Furthermore, in many countries, coagulase-negative staphylococci have become the major pathogenic agents responsible for mastitis found in pre- and postpartum milk samples (Same de Vliegher, Large Animal Review, 12, n. 6, 2006) and these microorganisms frequently present resistance to antibiotics and biocides.

Also, in several Countries, the use of antibiotics during dry off will be soon dismissed by law.

Various non-antibiotic intramammary antimicrobial products are available, however cure rates for clinical mastitis remain perplexingly low: 46% for *Streptococcus* spp., 21% for *Staphylococcus* spp., and only 9% for *Staphylococcus aureus* mastitis (See Wilson et al. (1996) National Mastitis Council Proceedings 164-165, and Crandall et al. (2005) NMC Annual Meeting Proceedings 215-216).

Therefore, it is highly desirable to provide new products for use in the treatment of any existing infection and/or prevention of new infections, in particular udder infections and mastitis, in the dairy animals.

Moreover, antibiotic treatments alone do not prevent new infections later on in the dry period due to the limited coverage of antibiotics.

Therefore, it is also highly desirable to provide new products for use in the treatment of any existing infection and/or prevention of new infections in the dairy animals, capable of maintaining sufficient coverage during the dry period.

Accordingly, it is an object of the present invention to provide a new composition for use as udder's antimicrobic treatment of dairy animals during the dry-off period.

### SUMMARY OF THE INVENTION

The above object has been achieved by a topical composition comprising a) at least one terpenoid, and b) at least one monoglyceride, in c) a fatty matrix, as claimed in claim 1. In a further aspect, the present invention also concerns the use of said composition in the udder's antimicrobic treatment in dairy animals, during the dry-off period, preferably by intramammary administration.

The characteristics and the advantages of the present invention will become clear from the following detailed description, and the working examples provided for illustrative purposes.

### DETAILED DESCRIPTION OF THE INVENTION

The present invention therefore relates to a veterinary topical composition, especially for diary animals, comprising:
a) at least one terpenoid selected from monoterpenoids, sesquiterpenoids, diterpenoids, and mixtures thereof, and
b) at least one monoglyceride selected from monoglycerides of C4-C23 fatty acids and mixtures thereof,
   in c) a fatty matrix comprising at least one medium chain triglyceride.

With the term "dairy animals", in the present invention, it is meant to include non-human animals for milk production. Dairy animals are preferably cattle, sheep, goats, buffaloes, camels, yaks, horses, reindeers and donkeys; more preferred dairy animals are cattle, sheep and goats.

Terpenoids, also known as isoprenoids, are a large and diverse class of naturally occurring organic chemicals derived from the 5-carbon compound isoprene, and the isoprene polymers called terpenes. While sometimes erroneously used interchangeably with "terpenes", indeed terpenoids contain additional functional groups, usually containing oxygen.

The composition of the invention comprises a) at least one terpenoid selected from monoterpenoids, sesquiterpenoids, diterpenoids, and mixtures thereof.

Monoterpenoids comprise bornyl acetate, camphor, carvone, citral, citronellal, citronellol, geraniol, eucalyptol, hinokitiol, iridoids, linalool, menthol, thymol, and mixtures thereof.

Sesquiterpenoids comprise farnesol, geosmin, humulone, and mixtures thereof.

Diterpenoids comprise resin acid, ginkgolides, paclitaxel, retinol, salvinorin A, sclareol, steviol, and mixtures thereof.

Preferably, the composition of the invention comprises a) at least one terpenoid selected from monoterpenoids, resin acid, and mixtures thereof.

For the purposes of the present invention, with the term "resin acid", it is meant to include abietic acid, dihydroabietic acid, dehydroabietic acid, neoabietic acid, palustric acid, pimaric acid, isopimaric acid, sandaropimaric acid, and mixtures thereof.

In preferred embodiments, said "resin acid" comprises a mixture of abietic acid, dehydroabietic acid, neoabietic acid, palustric acid and pimaric acid. More preferably, said "resin acid" comprises at least 80wt% of a mixture of abietic acid, dehydroabietic acid, neoabietic acid, palustric acid and pimaric acid, based on the weight of resin acid. In preferred embodiments, the composition of the invention comprises a) resin acid and at least one monoterpenoid. In these embodiments, said resin acid is preferably provided as rosin comprising the same.

Rosin, also known as Colophony, Greek pitch, violin resin, or rubber resin, is a vegetal resin that has a solid consistency and a yellow ochre colour, transparent. This resin is obtained from pines and other plants, mostly conifers, produced by heating fresh liquid resin to vaporize the volatile liquid terpene components.

From the chemical point of view, rosin comprises up to 99% of resin acids and neutral matter.

Depending on the origin, different types of industrial rosin products can be distinguished. The three main types of rosin products are Tall Oil Rosin (TOR), Wood Rosin and GUM Rosin. TOR is a by-product of wood pulping in the kraft process: it is the resin acid fraction separated by vacuum distillation from Crude Tall Oil (CTO) which is produced by the preparation of pulp. CTO is obtained via acidulation of Crude Tall Oil Soap or Crude Sulphate Soap (TOS). TOS is separated from cooking liquid in pulp mill often called black liqueur during pulping process.

Wood Rosin is the fraction separated by steam distillation or other means from dead trees, tree stumps, branches etc. and GUM Rosin is the non-volatile residue that has been steam distilled or separated by other means from tree resin harvested by tapping from a living tree.

Substances containing resin acids and obtained by vacuum distillation from crude tall oil include Distilled Tall Oil (DTO), Tall Oil Fatty Acid (TOFA) and Tall Oil Pitch (TOP). DTO contains 10-40% of resin acids. CTO typically contains 15-70% of resin acids, and the lowest resin acid contents are generally provided by the cooking of mixed wood pulp. The term "Tall Oil Rosin" or "TOR" should be understood as referring to a composition obtained by distillation of crude tall oil and further refinement of distilled tall oil.

TOR typically comprises 60-99% (w/w) resin acids.

The term "Wood Rosin" should be understood as referring to a composition obtained by distillation or other means from dead trees, tree stumps, branches etc. Wood Rosin typically comprises 50-99% (w/w) resin acids.

The term "GUM Rosin" should be understood as referring to a composition obtained by distillation or separated by other means from resin harvested from a living tree. GUM Rosin typically comprises 50-99% (w/w) resin acids.

The term "Distilled Tall Oil" or "DTO" should be understood as referring to a composition obtained by distillation of crude tall oil and further refinement of distilled tall oil. DTO typically comprises 10-60% (w/w) resin acids.

The resin acids are preferably present in the composition of the invention as TOR, Wood Rosin, GUM Rosin, CTO, TOS, DTO or mixtures thereof, more preferably as TOR, Wood Rosin and GUM Rosin.

Preferably, the composition of the invention comprises a) up to 20wt% of at least one terpenoid, based on the composition weight. If component a) comprises resin acid, the composition of the invention comprises a) up to 20wt% of resin acid, or rosin or a rosin product in a weight amount so as to provide up to 20wt% of resin acid, based on the composition weight.

More preferably, the composition of the invention comprises a) 1-20wt% of at least one terpenoid, even more preferably 5-18wt%, based on the composition weight.

The composition of the invention also comprises b) at least one monoglyceride selected from monoglycerides of C4-C22 fatty acids and mixtures thereof.

Monoglycerides (also known as acylglycerols or monoacylglycerols) are a class of glycerides which are composed of a molecule of glycerol linked to a fatty acid via an ester bond.

Said monoglycerides of C4-C22 fatty acids are monoglycerides of saturated and unsaturated C4-C22 fatty acids, such as butyric acid, isobutyric acid, valeric acid, isovaleric acid, 2-methylbutyric acid, caprylic acid, capric acid, lauric acid, myristic acid, palmitic acid, stearic acid, arachidic acid, behenic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, docosahexaenoic acid.

In preferred embodiments, said monoglycerides of C4-C22 fatty acids are monoglycerides of saturated C4-C22 fatty acids.

More preferably, the component b) comprises at least one monoglyceride selected from monoglycerides of C4-C18 fatty acids and mixtures thereof.

In the most preferred embodiments, the component b) comprises a monoglyceride of C4 fatty acid (i.e. glycerol monobutyrate or monobutyrin), a monoglyceride of C12 fatty acid (i.e. glycerol monolaurate or monolaurin) or a monoglyceride of C16 fatty acid (i.e. glycerol monostearate or monostearin), or a mixture thereof.

Preferably, the composition of the invention comprises b) up to 20wt% of at least one monoglyceride, based on the composition weight.

More preferably, the composition of the invention comprises b) 1-20wt% of at least one monoglyceride, even more preferably 5-18wt%, based on the composition weight.

In preferred embodiments, the component a) and the component b) are present in the composition in a weight ratio of 1:3 to 3:1, more preferably 1:2 to 2:1.

According to the present invention, the components a) and b) are in c) a fatty matrix comprising at least one medium chain triglyceride.

Medium-chain triglycerides (MCTs) are triglycerides with three fatty acids having an aliphatic tail of 6-12 carbon atoms, i.e., medium-chain fatty acids (MCFAs) or C6-C12 fatty acids, such as caproic acid, caprylic acid, capric acid, lauric acid.

Preferably, said at least one medium chain triglyceride is C8 fatty acid triglyceride, C10 fatty acid triglyceride, or a mixture thereof.

In preferred embodiments, said at least one medium chain triglyceride is a mixture of C8 fatty acid triglyceride, C10 fatty acid triglyceride, and traces of C6 and C12 fatty acid triglycerides.

In preferred embodiments, the components a) and b), as taken together, and c) a fatty matrix are in a weight ratio of 1:5 to 1:1, more preferably 1:3 to 1:1.5.

Preferably, the c) fatty matrix further comprises a lubricant selected from vegetable oil, vegetable gel, olein, natural or synthetic wax, spermaceti, lanolin, lanolin alcohol, paraffin, petrolatum, drying oil, and mixtures thereof.

Suitable vegetable oils are selected from neem oil, soybean oil, coconut oil, rapeseed (canola) oil, peanut oil, crambe oil, sunflower oil, karanja oil, neem oil, rice-bran oil, castor oil, sesame oil, rosehips oil, almond oil, raspberry oil, jojoba oil, avocado oil, moringa oil, olive oil, grape seed oil, argan oil, baobab oil, corn oil, Eucommia ulmoides seed oil, Sumac fruit oil, Kenaf seed oil, yellow horn seed oil, walnut oil, Sacha inchi oil, tigemut oil, hazelnut oil, Swida wilsoniana oil, Suaeda salsa seed oil, and combinations thereof.

Vegetable oils are the richest sources of vitamin E.

Tocopherols and tocotrienols, collectively referred to as tocols or vitamin E, are a family of naturally occurring fat-soluble compounds, which include eight members (alpha-, beta-, gamma-, and delta-tocopherol (T); and alpha-, beta-, gamma-, and delta-tocotrienol (T3)).

Vitamin E is an integral part of the skin's antioxidant defenses, primarily providing protection against UV radiation and other free radicals that may come in contact with the epidermis. Oral supplementation with only vitamin E may not provide adequate protection for the skin, and co-supplementation of vitamin E and vitamin C may be warranted to effectively increase the photoprotection of skin through the diet. However, topical vitamin E seems to be an effective mechanism for both delivery to the skin and providing a photoprotective effect. Additional anti-inflammatory effects of topical vitamin E have been seen in the skin, although more studies are needed to determine if vitamin E primarily works as a free-radical scavenger or can have other effects on inflammatory signaling. In any case, for the purposes of the present invention, when said lubricant selected from vegetable oils, the composition achieves an additional advantage of providing Vitamin E.

Suitable vegetable gels are aloe vera gel, carrageenan gel, algae gel, and combinations thereof.

More preferably, said c) fatty matrix further comprises a lubricant selected from vegetable oil, vegetable gel, petrolatum, or a mixture thereof.

In preferred embodiments, said c) fatty matrix further comprises petrolatum. Preferably, the composition of the invention comprises c) 90-30wt% of a fatty matrix, more preferably 75-40wt%, based on the composition weight.

More preferably, said c) fatty matrix comprises at least one medium chain triglyceride and a lubricant.

In preferred embodiments, in the c) fatty matrix, said at least one medium chain triglyceride and said lubricant are in a weight ratio of 1:5 to 1:1, more preferably 1:3 to 1:1.5.

In preferred embodiments, the composition of the invention has a Brookfield viscosity of 80 to 200 Pa*s at T=25°C. The viscosity of the composition is measured by using a viscometer. The preferred viscometer is Brookfield and rotating viscometer. At these viscosities, the composition of the invention shows optimal grip and adhesion properties once applied to the skin. This means that the composition is advantageously in contact with the skin for periods of time suitable for achieving the desired effect on the skin. More preferably, the composition of the invention has a Brookfield viscosity of 100 to 170 Pa*s at T=25°C, RV06-RPM05, even more preferably, 120 to 150 Pa*s at T=25°C, RV06-RPM05, where RV is one of the four basic spring torque series offered by Brookfield and RPM is round per minute.

It should be appreciated that the composition of the invention needs no additional active principles, such as drugs or hormones. Therefore, the composition of the invention is free of drugs and hormones.

Preferably, the composition of the invention further comprises d) at least one anti-oxidant agent, more preferably selected from vitamin A, vitamin C, vitamin E, beta-carotene, lycopene, lutein, selenium, and mixtures thereof.

It should be understood that said d) at least one anti-oxidant agent is present in addition to the anti-oxidants naturally occurring in the other components a) to c), with the aim to increase their overall content in the composition of the invention.

In preferred embodiments, said at least one anti-oxidant agent is a vitamin.

In more preferred embodiments, said vitamin is vitamin E.

If the c) fatty matrix further comprises a lubricant selected from vegetable oils, vitamin E is optionally further added, with the aim to increase the overall vitamin E content in the composition of the invention.

With "vitamin E" is preferably meant a vitamin E salt or a vitamin E ester, such as vitamin E acetate or vitamin E phosphate.

In preferred embodiments, the composition of the invention further comprises 1-15wt% of Vitamin E, based on the composition weight.

Preferably, the composition of the invention further comprises e) a lignin fraction, said lignin fraction comprising fragments having a weight average molecular weight of 2,000-8,000 Daltons, as measured by Size-Exclusion Chromatography, said fragments comprising 11 to 44 phenylpropane units on weight average.

Lignin is a class of complex organic polymers. Chemically, lignin is a very irregular, randomly cross-linked polymer of phenylpropane units joined by many different linkages, with a weight average molecular weight of 20,000 Daltons or higher. Lignin can be categorized to softwood and hardwood lignin according to the raw biomass sources. Suitable starting materials for obtaining the relevant lignin fraction are any lignin including essentially pure lignin as well as kraft lignin, biomass originating lignin, lignin from alkaline pulping process, lignin from soda process, lignin from organosolv pulping and any combination thereof.

By the expression "essentially pure lignin", it is to be understood an at least 90% pure lignin on a dry raw biomass basis, preferably at least 95% pure lignin, the remainder being extractives and carbohydrates such as hemicelluloses as well as inorganic matter.

By the expression "kraft lignin", it is to be understood lignin that originates from kraft black liquor. For the purposes of the present invention, lignin fraction can be obtained by any suitable method, as described in WO2018001935.

For the purposes of the present invention, the weight average molecular weight (M_{w}) of fragments in the lignin fraction is measured by Size-Exclusion Chromatography (or 'SEC'). In the present invention, the eluent is preferably 0.1 M NaOH.

The molecular weight of the three phenylpropanoid monomer precursors varies between 150 Da of coumaryl alcohol, 180 Da of coniferyl alcohol, and 210 Da of synapyl alcohol. The average weight is therefore 180 Da and this value has been used as "phenylpropane unit". The M_{w} values have been divided by 180 Da, thus obtaining the phenylpropane unit numbers on weight average.

Preferably, said e) lignin fraction comprises fragments having a weight average molecular weight of 3,000-6,000 Daltons, as measured by Size-Exclusion Chromatography, said fragments comprising 16 to 33 phenylpropane units on weight average.

Without wishing to be bound by any theory, it is believed that lower number average molecular weights mean more active molecules. This is put forward considering that lower molecular weights mean smaller fragments, and smaller fragments mean less crosslinked/shorter fragments, and less crosslinked/shorter fragments mean a higher number of free functional groups thereon, thus more reactive fragments.

In further embodiments, the lignin fraction has a polydispersity index (PDI) of 1.25 to 6. The polydispersity index (PDI) or heterogeneity index, or simply dispersity, is a measure of the distribution of molecular mass in a given polymer sample. PDI is the weight average molecular weight (M_{w}) divided by the number average molecular weight (Mₙ). It indicates the distribution of individual molecular masses in a batch of polymers. Preferably the lignin fraction is present in the composition in liquid form as a solution of lignin fraction and suitable solvent.

Suitable solvents are water, glycols, alcohols, polyalcohols, aldehydes, ketones, organic acids, DMSO, and combinations thereof. Preferred solvents are water, methanol, ethanol, n-propanol, iso-propanol, n-butanol, isobutanol, allyl alcohol, 1,2-propylene glycol, 1,3-propylene glycol, 1,2-ethylene glycol, polyethylene glycol (PEG), benzyl alcohol, glycerol, acetone, lactic acid, polylactic acid, DMSO, and mixtures thereof.

More preferably, the lignin fraction is present in the composition as a solution of lignin fraction in DMSO, most preferably of 25% (w/w) of lignin fraction and 75% (w/w) of DMSO, based on the total weight of the solution.

In preferred embodiments, the composition of the invention further comprises 5-20wt% of said e) lignin fraction, based on the composition weight.

Optionally, the composition of the invention further comprises 1-10wt% of zinc oxide, based on the weight of the composition.

Preferably, the veterinary topical composition comprises:
a) up to 20wt% of at least one terpenoid selected from monoterpenoids, sesquiterpenoids, diterpenoids, and mixtures thereof, and
b) up to 20wt% of at least one monoglyceride selected from monoglycerides of C4-C23 fatty acids and mixtures thereof,
   in c) 90-30wt% of a fatty matrix comprising at least one medium chain triglyceride, based on the composition weight.

More preferably, the veterinary topical composition comprises:
a) 1-20wt% of at least one terpenoid selected from monoterpenoids, sesquiterpenoids, diterpenoids, and mixtures thereof, and
b) 1-20wt% of at least one monoglyceride selected from monoglycerides of C4-C23 fatty acids and mixtures thereof,
   in c) 75-40wt% of a fatty matrix comprising at least one medium chain triglyceride, based on the composition weight.

It should be also understood that all the combinations of preferred aspects of the components a), b), and c) above described are likewise preferred and deemed to be hereby disclosed, also for these preferred embodiments of the veterinary topical composition. In other embodiments, the composition of the invention consists essentially of:
a) up to 20wt% of at least one terpenoid selected from monoterpenoids, sesquiterpenoids, diterpenoids, and mixtures thereof, and
b) up to 20wt% of at least one monoglyceride selected from monoglycerides of C4-C23 fatty acids and mixtures thereof,
d) at least one anti-oxidant agent; and
e) a lignin fraction, as above described,
in c) 90-30wt% of a fatty matrix comprising at least one medium chain triglyceride, based on the composition weight.

For the purposes of the present invention, the expression "consists essentially of" means that the ingredients a)-e) are the only active components which are present in the compositions, the possible other ingredients being simple co-formulants or excipients. In other embodiments, the composition of the invention consists of:
a) up to 20wt% of at least one terpenoid selected from monoterpenoids, sesquiterpenoids, diterpenoids, and mixtures thereof, and
b) up to 20wt% of at least one monoglyceride selected from monoglycerides of C4-C23 fatty acids and mixtures thereof,
d) at least one anti-oxidant agent; and
e) a lignin fraction, as above described,
in c) 90-30wt% of a fatty matrix comprising at least one medium chain triglyceride, based on the composition weight.

It should be also understood that all the combinations of preferred aspects of the components a), b), and c) above described are likewise preferred and deemed to be hereby disclosed, also for these preferred embodiments of the veterinary topical composition defined by the expression "consists essentially of" and "consists of".

Preferred compositions according to the invention are provided in the following tables, where "Rosin (Colophony)" comprises at least 80wt% of a mixture of abietic acid, dehydroabietic acid, neoabietic acid, palustric acid and pimaric acid, based on the weight of rosin, and where the active components are reported (where "wt%" means "wt% based on the composition weight"):
Composition 1 comprises:

| | **wt%** |
|---|---|
| Rosin (Colophony) | 20-25 |
| Monolaurin | 10-15 |
| Petrolatum | 25-30 |
| Medium Chain Triglycerides (MCTs) | 22-32 |

Composition 1A comprises:

| | **wt%** |
|---|---|
| Rosin (Colophony) | 12-18 |
| Monolaurin | 8-12 |
| Petrolatum | 23-27 |
| Medium Chain Triglycerides (MCTs) | 18-22 |
| Vitamin E | 13-17 |
| Lignin fraction-based solution (25 wt.% of lignin fraction in DMSO) | 13-17 |

Composition 2 comprises:

| | **wt%** |
|---|---|
| Rosin (Colophony) | 11-15 |
| Monolaurin | 10-14 |
| Vegetable oil | 35-45 |
| Medium Chain Tryglicerides (MCTs) | 18-22 |
| Lignin fraction-based solution (25 wt.% of lignin fraction in DMSO) | 13-17 |

Composition 3 comprises:

| | **wt%** |
|---|---|
| Rosin (Colophony) | 16-20 |
| Monolaurin | 13-17 |
| Aloe vera gel | 25-30 |
| Medium Chain Tryglicerides (MCTs) | 16-20 |
| vitamin E acetate | 8-12 |
| Lignin fraction-based solution (25 wt.% of lignin fraction - 75 wt.% DMSO) | 10-12 |

Composition 4 comprises:

| | **wt%** |
|---|---|
| Rosin (Colophony) | 10-15 |
| (-)-menthol | 2-7 |
| Monobutyrin | 13-17 |
| Petrolatum | 8-12 |
| Neem oil | 15-20 |
| Medium Chain Tryglicerides (MCTs) | 15-20 |
| vitamin E acetate | 8-12 |
| Lignin fraction-based solution (25 wt.% of lignin fraction - 75 wt.% DMSO) | 10-14 |

Composition 5 comprises:

| | **wt%** |
|---|---|
| Rosin (Colophony) | 13-17 |
| Monostearin | 5-10 |
| Vegetable oil | 40-50 |
| Medium Chain Tryglicerides (MCTs) | 18-22 |
| Lignin fraction-based solution (25 wt.% of lignin fraction - 75 wt.% DMSO) | 11-15 |

The composition of the present invention can be prepared by method known in the art, however a specific and preferred preparation has been conceived and designed to this end.

Preferably, the composition of the invention is prepared by a process comprising the following steps:
- heating and mixing the components a) - b) and the fatty matrix c), preferably at a temperature of 70-80°C;
- optionally adding vitamin E and the lignin fraction, and mixing in a turboemulsifier;
- cooling at a temperature below 30°C, thus obtaining the composition of the invention.

Preferably, the composition is then used to fill single-dose syringes (of about 10 mL of volume, corresponding to 8-10 mg of composition), ready for use.

In a further aspect, the present invention also concerns the veterinary topical composition of the invention for use in the antimicrobic treatment of dairy animals' udders. Preferably, said composition is to be administered during the dry-off period, preferably by intramammary administration.

With the term "antimicrobic treatment of dairy animals' udders", it is meant that the veterinary topical composition of the invention is suitable to be administered on or inside udders of dairy animals and capable of remaining on or inside the udders for a period of time sufficient to provide antimicrobic activity. The composition of the invention has both texture and efficacy that make it suitable for antimicrobic treatment of dairy animals' udders.

With the term "udder's antimicrobic treatment" or "antimicrobic treatment of udders", in the present invention, it is meant the treatment of infections of the udder of dairy animals. With "treatment of infections", it is meant either the prevention of new infections and the reduction or elimination of pre-existing infections, if present.

The most widely accepted criterion for indicating the udder health status of a dairy animal is the count of somatic cells per millilitre in a fluid specimen, usually milk. White blood cells (leukocytes) constitute the majority of the somatic cells in question. The number of somatic cells increases in response to pathogenic bacteria that can cause mastitis. Therefore, the somatic cells count (SCC) can be employed to assess efficacy of an udder's antimicrobic treatment, for example by counting somatic cells before and after treatment. General agreement rests on a reference range of less than 200,000 cells/mL for uninfected cows and greater than or equal to 250,000 cells/mL for cows infected with significant pathogen levels.

A low SCC, i.e., below 200,000 cells/mL, preferably below 100,000 cells/mL after calving and at recovery of lactation suggests that either the dry cow treatment cow effectively reduced any infection or prevented new infections during the dry period. An elevated SCC, i.e., equal to or over 200,000 cells/mL after calving and at recovery of lactation indicates that a new infection has developed.

If the SCC remains high from the last SCC in lactation through the first functional control (F.C.) in the next lactation, either the dry cow treatment was ineffective or the infection has walled itself off with scar tissue and became resistant to the treatment (G.M. Jones, Dairy Science 404-228, 1998*).*

Therefore, the udder's antimicrobic treatment according to the invention is effective when, in the majority of the animals of the herd, the SCC is maintained below 200,000 cells/mL after dry-off, preferably below 100,000 cells/mL (prevention of new infections or treatment of new infections occurring during dry-off), or when, in the animals having an SCC equal to or over 200,000 cells/mL before dry-off, the treatment reduces the SCC after dry-off, preferably below 200,000 cells/mL, more preferably below 100,000 cells/mL (treatment of pre-existing infections).

Typically, the udder health status of a dairy animal is also evaluated by assessing the presence of pathogenic microorganism in milk (bacteriology).

Therefore, the udder's antimicrobic treatment according to the invention is also effective when, in the majority of animals of the herd, the bacteriology after drying off is negative to pathogenic microorganisms and/or the presence of pathogenic microorganism is reduced compared to their presence before treatment.

Typical pathogenic microorganisms include gram-positive bacteria, such as: *Coagulase-negative Staphylococcus* (CoNS), *Corynebacterium spp., Enterococcus spp., Streptococcus uberis, Streptococcus dysgalactiae, Staphylococcus epidermidis, Klebsiella spp., Serratia marcescens,* among others.

The udder's antimicrobic treatment in dairy animals is aimed at preventing and/or treating infections of the udder, in particular mastitis.

Therefore, the present invention also concerns the use of the composition of the invention in the treatment of udder's infections in dairy animals, in particular of mastitis. Conveniently, the composition of the invention is particularly suitable for intramammary administration, especially owing to its viscosity, as it can be advantageously introduced in the udder quarters of dairy animals and it is maintained in the udder for a time sufficient to provide the desired antimicrobic action.

As it will be widely discussed and demonstrated in the Examples below, the association of terpenoids and monoglycerides of fatty acids, in a fatty matrix, provides an effective antimicrobic action that can prevent and/or treat infections in dairy animals during the critical dry-off period.

Surprisingly and unexpectedly, the efficacy of the composition is comparable to the efficacy of antibiotics. Therefore, the composition of the invention provides an effective treatment that can substitute the use of antibiotics during dry-off

Without being bound to theory, it is believed that the combination of the claimed components in the composition of the invention impart also desirable physical features to the composition itself, such a density at room or body temperature that is suitable to have the composition be maintained on the udder and/or inside the udder for a sufficient amount of time for performing its antimicrobic function during all or almost all the dry-off period. In particular, it is believed that the overall lipophilic nature of the composition improves the maintenance of the same on the udder and/or inside the udder, even in case of milk leakage.

Thus preferably, the composition of the invention is in the form of a gel, more preferably for intramammary administration.

In further preferred embodiments, the composition of the invention is in the form of a cream or spray formulation. Said cream or spray formulations preferably further comprise water, film-forming agents, alcohols, polyols and/or glycols. Cream formulations preferably comprise less than 50wt% of hydrocarbons, waxes, or polyethylene glycols. Optionally, the formulation comprising the composition of the invention, is an oil in water (O/W) formulation.

Cream or spray formulations are particularly suitable for topical administration on the udders of animals.

Preferably, the composition of the invention in the form of gel is administered intramammary at the beginning of the dry off period. Optionally, the same composition in the form of cream or spray is administered topically on the quarters at least once during the dry off period.

A suitable dosage is to provide up to 10 ml of composition in each udder quarter of the animal. Typically, the composition is provided in single-doses of 10 ml, for administration to each quarter of the udder.

It should be also understood that all the combinations of preferred aspects of the composition of the invention, formulations and uses of the same, as above reported, are to be deemed as hereby disclosed.

Below are working examples of the present invention provided for illustrative purposes.

### EXAMPLES

### Example 1

The compositions reported in the table below, have been prepared as follows:
Rosin, monolaurin and Vitamin E in the fatty matrix are mixed at 70-80°C obtaining a uniform solution.

Lignin fraction-based solution is then added by vigorous mixing in a turboemulsifier, then the composition thus obtained is quickly cooled at a temperature below 30°C to obtain a stable gel.

| | **Ex. 1** | **Ex. 1A** |
|---|---|---|
| | **wt%** | **wt%** |
| Rosin (Colophony)* | 20 | 15 |
| Monolaurin | 15 | 10 |
| White Petrolatum | 35 | 25 |
| Medium Chain Tryglicerides (MCTs) | 30 | 20 |
| vitamin E acetate | - | 15 |
| Lignin fraction-based solution (25 wt.% of lignin fraction - 75 wt.% DMSO) | - | 15 |
| Brookfield viscosity (T=25°C; RV06 RPM05) | 145.4 Pa*s | 136.8 Pa*s |

| | | |
|---|---|---|
| * comprising about 95wt% of a mixture of abietic acid, dehydroabietic acid, neoabietic acid, palustric acid and pimaric acid | | |

### Example 2

The composition below has been prepared as described in example 1:

| | **wt%** |
|---|---|
| Rosin (Colophony)* | 15 |
| Monolaurin | 10 |
| Neem oil | 25 |
| Medium Chain Tryglicerides (MCTs) | 20 |
| vitamin E acetate | 15 |
| Lignin fraction-based solution (25 wt.% of lignin fraction - 75 wt.% DMSO) | 15 |
| Brookfield viscosity (T=25°C; RV06 RPM05) | 140.3 Pa*s |

| | |
|---|---|
| * comprising about 85wt% of a mixture of abietic acid, dehydroabietic acid, and neoabietic acid | |

### Example 3

The composition below has been prepared as described in example 1:

| | **wt%** |
|---|---|
| Rosin (Colophony)* | 18 |
| Monobutyrin | 15 |
| Aloe vera gel | 35 |
| Medium Chain Tryglicerides (MCTs) | 20 |
| Lignin fraction-based solution (25 wt.% of lignin fraction - 75 wt.% DMSO) | 12 |
| Brookfield viscosity (T=25°C; RV06 RPM05) | 125.8 Pa^{∗}s |

| | |
|---|---|
| * comprising about 98wt% of a mixture of abietic acid, dehydroabietic acid, neoabietic acid, palustric acid and pimaric acid | |

### Example 4

Efficacy of the composition of example 1, according to a preferred embodiment of the invention (*"Inv. Comp."*)*,* has been tested in a first group of cows, treated intramammary at the beginning of dry off (10 ml of the composition/quarter).

A second group of cows has been treated intramammary with antibiotics at the beginning of dry off, for comparison.

Dry off in the two groups of cows has been interrupted 55-70 days before calving.

### Example 5

Bacteriology has been performed in milk of the cows of the first group of Example 4 (treated with the composition of the invention), before dry off and after calving.

The results are provided in table below:

| **Bacteriology at dry off** | **Bacteriology Post Partum** |
|---|---|
| *St. Coag -* | *Neg* |
| *St. Coag -* | *Neg* |
| *St. Coag -* | *Neg* |
| *St. Coag -* | *Neg* |
| - | *Neg* |
| *St. Coag -* | *Neg* |
| *Corynebacterium spp.* | *Neg* |
| *Neg* | *St. Coag-* |
| *St. Coag -* | *Neg* |
| *Neg* | *Neg* |

Except for only one cow, surprisingly the cows that have received the composition of the invention do not show any presence of pathogenic microorganisms after calving. In particular, the results show efficacy of the composition of the invention in treating infections that were present before dry off.

### Example 6

Somatic Cells Count (SCC) has been performed in milk of all the cows of example 4, before dry off: three Functional Control, F.C., were performed at 3, 2 and 1 month before dry off (F.C. -3, -2 and -1) and after calving (1^{st} F.C. post partum).

The results are provided in Tables A (cows treated with the composition of the invention) and B (cows treated with antibiotics). Data are shown as number of cells/ml.

**Table A**

| Treatment | **ID Cow** | **F.C.** - **3** | **F.C.** - **2** | **F.C.** - **1** | **1^{st} F.C. Post Partum** |
|---|---|---|---|---|---|
| *Inv. Comp.* | 5323 | 24.000 | 22.000 | 27.000 | 208.000 |
| *Inv. Comp* | 5346 | 27.000 | | 10.000 | 3.000 |
| *Inv. Comp* | 8140 | 58.000 | 53.000 | 129.000 | 12.000 |
| *Inv. Comp* | 8033 | 68.000 | 85.000 | 202.000 | 8.000 |
| *Inv. Comp* | 8070 | 73.000 | 58.000 | 170.000 | 57.000 |
| *Inv. Comp* | 5345 | 78.000 | 89.000 | 66.000 | 31.000 |
| *Inv. Comp* | 9640 | 122.000 | 52.000 | 296.000 | 387.000 |
| *Inv. Comp* | 9759 | 282.000 | 254.000 | 205.000 | 220.000 |
| *Inv. Comp* | 9570 | 297.000 | 287.000 | 384.000 | 24.000 |
| *Inv. Comp* | 9719 | 759.000 | 589.000 | 780.000 | 181.000 |

**Table B**

| **Treatment** | **ID Cow** | **F.C. - 3** | **F.C. - 2** | **F.C. - 1** | **1^{st} F.C. Post Partum** |
|---|---|---|---|---|---|
| *Cefatron A* | 9777 | 42.000 | 91.000 | 490.000 | 11.000 |
| *Mamyzin* | 9758 | 44.000 | 531.000 | 89.000 | 3.000 |
| *Cefatron A* | 5328 | 65.000 | 404.000 | 239.000 | 102.000 |
| *Cefatron A* | 5335 | 66.000 | 219.000 | 280.000 | 19.000 |
| *Cefatron A* | 5382 | 101.000 | 1.586.000 | 42.000 | 23.000 |
| *Cefatron A* | 9700 | 176.000 | 730.000 | 516.000 | 8.000 |
| *Cefatron A* | 5410 | 276.000 | 414.000 | 21.000 | 37.000 |
| *Cefatron A* | 5379 | 658.000 | 393.000 | 1.962.000 | 58.000 |
| *Cefatron A* | 7837 | 1.870.000 | 215.000 | 179.000 | 1.342.000 |

The somatic cell count confirmed the efficacy of the composition of the invention. Moreover, its efficacy is almost comparable to the efficacy of antibiotics.

### Example 7

Efficacy of the composition of Example 3 (*"Inv. Comp."*) has been tested in a first group of cows treated intramammary at the beginning of dry off (10 ml of the composition/quarter).

A second group of cows was not treated with any antimicrobic agent, but was sealed at dry off.

### Example 8

Somatic Cells Count (SCC) has been performed in milk of all the cows of example 7, at the beginning and at the end of dry off.

The results are provided in Tables C (cows treated with the composition of the invention) and D (cows sealed and non-treated). Data are shown as number of cells/ml.

**Table C**

| Treatment | **SCC at DRY OFF start** | **SCC at DRY OFF end** |
|---|---|---|
| *Inv. Comp.* | 112.000 | 52.000 |
| *Inv. Comp.* | 131.000 | 52.000 |
| *Inv. Comp.* | 202.000 | 77.000 |
| *Inv. Comp.* | 44.000 | 172.000 |
| *Inv. Comp.* | 207.000 | 79.000 |
| *Inv. Comp.* | 543.000 | 111.000 |
| *Inv. Comp.* | 137.000 | 54.000 |
| *Inv. Comp.* | 459.000 | 385.000 |
| *Inv. Comp.* | 1.200.000 | 301.000 |
| *Inv. Comp.* | 144.000 | 29.000 |
| *Inv. Comp.* | 2.445.000 | 153.000 |
| *Inv. Comp.* | 77.000 | 228.000 |

**Table D**

| Treatment | **SCC at DRY OFF start** | **SCC at DRY OFF end** |
|---|---|---|
| *only seal* | 30.000 | 24.000 |
| *only seal* | 26.000 | 15.000 |
| *only seal* | 175.000 | 223.000 |
| *only seal* | 89.000 | 188.000 |
| *only seal* | 186.000 | 1.924.000 |
| *only seal* | 37.000 | 1.006.000 |
| *only seal* | 150.000 | 132.000 |
| *only seal* | 164.000 | 105.000 |
| *only seal* | 128.000 | 112.000 |
| *only seal* | 15.000 | 20.000 |
| *only seal* | 64.000 | 1.206.000 |
| *only seal* | 223.000 | 82.000 |

Whit the seal alone, the majority of cows show a SCC (much) higher than 200.000 at the end of dry off, which is a signature of infection by pathogenic microorganisms.

Surprisingly, in cows treated with the composition of the invention, the majority of animals show a SCC below 200.000 at the end of dry off (many even below 100.000). Moreover, a strong reduction of the SCC in cows that presented a high SCC at the beginning of dry off has been demonstrated after treatment with the composition of the invention.

## Claims

1. A veterinary topical composition, especially for dairy animals, comprising:
a) at least one terpenoid selected from monoterpenoids, sesquiterpenoids, diterpenoids, and mixtures thereof, and
b) at least one monoglyceride selected from monoglycerides of C4-C23 fatty acids and mixtures thereof,
in c) a fatty matrix comprising at least one medium chain triglyceride.

2. The composition of claim 1, wherein said a) at least one terpenoid is selected from monoterpenoids, abietic acid, and mixtures thereof.

3. The composition of claim 1 or 2, wherein said monoglycerides of C4-C22 fatty acids are monoglycerides of saturated C4-C22 fatty acids.

4. The composition of any one of claims 1-3, wherein the component a) and the component b) are in a weight ratio of 1:3 to 3:1, preferably 1:2 to 2:1.

5. The composition of any one of claims 1-4, wherein, in said c) fatty matrix, said at least one medium chain triglyceride is C8 fatty acid triglyceride, C10 fatty acid triglyceride, or a mixture thereof.

6. The composition of any one of claims 1-5, wherein the components a) and b), as taken together, and c) the fatty matrix are in a weight ratio of 1:5 to 1: 1, preferably 1:3 to 1:1.5.

7. The composition of any one of claims 1-6, wherein the c) fatty matrix further comprises a lubricant selected from vegetable oil, vegetable gel, olein, natural or synthetic wax, spermaceti, lanolin, lanolin alcohol, paraffin, petrolatum, drying oil, and mixtures thereof.

8. The composition of any one of claims 1-7, further comprising d) at least one anti-oxidant agent, preferably selected from vitamin A, vitamin C, vitamin E, beta-carotene, lycopene, lutein, selenium, and mixtures thereof.

9. The composition of any one of claims 1-8, further comprising e) a lignin fraction, said lignin fraction comprising fragments having a weight average molecular weight of 2,000-8,000 Daltons, as measured by Size-Exclusion Chromatography, said fragments comprising 11 to 44 phenylpropane units on weight average.

10. The composition of any one of claims 1-9, comprising:
a) up to 20wt% of at least one terpenoid selected from monoterpenoids, sesquiterpenoids, diterpenoids, and mixtures thereof, and
b) up to 20wt% of at least one monoglyceride selected from monoglycerides of C4-C23 fatty acids and mixtures thereof,
in c) 90-30wt% of a fatty matrix comprising at least one medium chain triglyceride, based on the composition weight.

11. The composition of claim 10 comprising:
a) 1-20wt% of at least one terpenoid selected from monoterpenoids, sesquiterpenoids, diterpenoids, and mixtures thereof, and
b) 1-20wt% of at least one monoglyceride selected from monoglycerides of C4-C23 fatty acids and mixtures thereof,
in c) 75-40wt% of a fatty matrix comprising at least one medium chain triglyceride, based on the composition weight.

12. The composition of any one of claims 1-11, having a Brookfield viscosity of 80 to 200 Pa*s at T=25°C, preferably a Brookfield viscosity of 100 to 170 Pa*s at T=25°C, RV06-RPM05, more preferably, 120 to 150 Pa*s at T=25°C, RV06-RPM05.

13. The composition of any one of claims 1-12, for use in the antimicrobic treatment of udders of dairy animals.

14. The composition for use of claim 13, for use in the treatment of mastitis of dairy animals.

15. The composition for use of claim 13 or 14, to be administered topically intramammary, or topically on the udders of the dairy animals, preferably during the dry off period, more preferably at the beginning of the dry off period.
